(19) Europäisches Patentamt
European Patent Office
Office européen des brevets

(11) Veröffentlichungsnummer: **0 290 630 A1**

(12) # EUROPÄISCHE PATENTANMELDUNG

Veröffentlicht nach Art. 158 Abs. 3 EPÜ

(21) Anmeldenummer: **88900265.5**

(22) Anmeldetag: **23.11.87**

Daten der zugrundeliegenden internationalen Anmeldung:

(86) Internationale Anmeldenummer:
**PCT/SU87/00135**

(87) Internationale Veröffentlichungsnummer:
**WO88/03923 (02.06.88 88/12)**

(51) Int. Cl.[3]: **C 07 D 473/18**
A 61 K 31/52

(30) Priorität: **25.11.86 SU 4152348**

(43) Veröffentlichungstag der Anmeldung:
**17.11.88 Patentblatt 88/46**

(84) Benannte Vertragsstaaten:
**DE FR GB IT**

(71) Anmelder: **INSTITUT ORGANICHESKOGO SINTEZA AKADEMII NAUK LATVIISKOI SSR**
**ul. Aizkraukles, 21**
**Riga 226006(SU)**

(71) Anmelder: **BELORUSSKY NAUCHNO-ISSLEDOVATELSKY INSTITUT EPIDEMIOLOGII I MIKROBIOLOGII**
**ul. Nogina, 3**
**Minsk, 220050(SU)**

(72) Erfinder: **ZHUK, Regina Abramovna**
**ul. Gorkogo, 77-20**
**Riga, 226013(SU)**

(72) Erfinder: **LIDAK, Marger Jurievich**
**ul. Mezhotnes, 37-1**
**Riga, 226002(SU)**

(72) Erfinder: **MADRE, Marina Anatolievna**
**ul. Lapu, 8-28**
**Ogre, 228300(SU)**

(72) Erfinder: **VOTYAKOV, Veniamin Iosifovich**
**ul. Myasnikova, 78-29**
**Minsk, 220050(SU)**

(72) Erfinder: **ANDREEVA, Olga Trifonovna**
**pr. Pushkina, 13-156**
**Minsk, 220015(SU)**

(72) Erfinder: **BOREKO, Evgeny Ivanovich**
**2-i per. Bagrationa, 19-765**
**Minsk, 220037(SU)**

(72) Erfinder: **KOROBCHENKO, Ljudmila Viktorovna**
**ul. Gorkogo, 64/1-87**
**Minsk, 220123(SU)**

(72) Erfinder: **RUSYAEV, Vyacheslav Anatolievich**
**ul. Odoevskogo, 103-2**
**Minsk, 220015(SU)**

(72) Erfinder: **STARKOVA, Olga Ivanovna**
**ul. Kr.Barona, 36-17**
**Riga, 226011(SU)**

(74) Vertreter: **von Füner, Alexander, Dr. et al,**
**Patentanwälte v. Füner, Ebbinghaus, Finck Mariahilfplatz 2 & 3**
**D-8000 München 90(DE)**

(54) **9-SUBSTITUIERTE GUANINE.**

(57) Neue Verbindungen 9-substituierte Guanine weisen folgende Formel

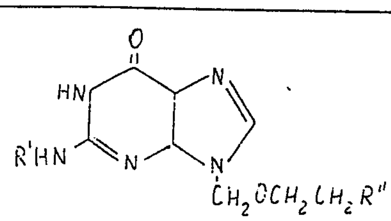

./...

**EP 0 290 630 A1**

auf, worin R' für Azetyl, Wasserstoff und R" für 2-Tetrahydrofuryl, 2-Tetrahydropyranyl stehen.

Die erfindungsgemäßen Verbindungen besitzen eine antivirale Aktivität.

# 9-SUBSTITUIERTE GUANINE

## Gebiet der Technik

Die vorliegende Erfindung bezieht sich auf die organische Chemie und betrifft insbesondere neue Verbindungen und zwar 9-substituierte Guanine. Die angegebenen Verbindungen weisen eine antivirale Aktivität auf.

## Verangehender Stand der Technik

Bekannt sind verschiedene Verbindungen, die gegen Viren wirksam sind, beispielsweise 9-(2-Hydroxyäthoxymethyl)-guanin der folgenden Formel

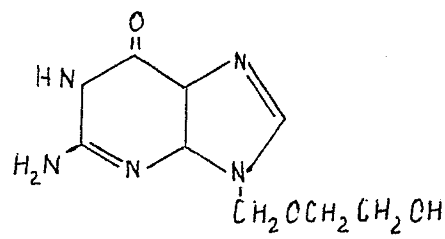

welches als Wirkstoff des Arzneimittels Zovirax (Azyklovir), hergestellt von der Firma "The Wellcome Foundation Ltd.", London (DE, A, Nr. 2539963) dient.

Zovirax ist ein effektives gegen Viren wirkendes Präparat der zweiten Generation, das Herpes simplex-Viren HSV-1 und HSV-2 sowie Zoster-Viren und andere Viren beim Menschen und Tier inaktiviert.

Das Präparat ist wenig wasserlöslich (2 mg/ml), und dadurch ist die Nephrotoxizität bei peroraler Applikation desselben zu vermerken.

Das Präparat wird intravenös als längere (zweistündige) Infusionen verabfolgt. Wegen kleiner Löslichkeit kann das Präparat intramuskulär nicht appliziert werden, und bei zufälliger intravenöser Verabreicherung ruft das in die umgebenden Gewebe gelangte Präparat ihre Nekrose wegen hoher alkalischer Reaktion der Injektionslösung (pH=11,0) hervor.

## Offenbarnung der Erfindung

Die beanspruchten Verbindungen sind neu gewonnen und in der Fachliteratur nicht beschrieben.

Der Erfindung liegt die Aufgabe zugrunde, neue

Verbindungen herzustellen, welche eine antivirale Aktivität besitzen und eine erhöhte Löslichkeit in Wasser und lipophilen Substanzen des Organismus aufweisen.

Die Aufgabe ist dadurch gelöst, daß die beanspruchten neuen Verbindungen 9-substituierte Guanine erfindungsgemäß die allgemeine Formel

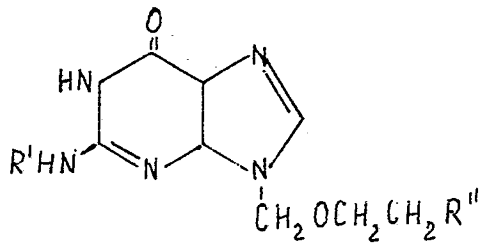

haben, worin

R' für Azetyl, Wasserstoff,

R'' für 2-Tetrahydrofuryl oder 2-Tetrahydropyranyl

stehen.

Die erfindungsgemäßen Verbindungen sind kristalline Substanzen, deren Struktur durch die Gesamtheit der physikalisch-chemischen Eigenschaften nachgewiesen wird (Tabelle 1 und 2).

Tabelle 1. Physikalisch-chemische Eigenschaften der erfindungsgemäßen Verbindungen

| Verbindung | $T_{Schmelz}$ in °C | $R_f$ im System Chloroform-Methanol = 10:2 | Gefunden in % C | H | N |
|---|---|---|---|---|---|
| 1 | 2 | 3 | 4 | 5 | 6 |
| 9-/2-(2-Tetrahydrofuryloxy)äthoxymethyl/- $N^2$ -azetylguanin | 162...163 | 0,63 | 49,6 | 5,6 | 20,7 |
| 9-/2-(2-Tetrahydrofuryloxy)äthoxymethyl/-guanin | 192-194 | 0,39 | 46,2 | 6,1 | 22,3 |

Fortsetzung der Tabelle 1

| 1 | 2 | 3 | 4 | 5 | 6 |
|---|---|---|---|---|---|
| 9-/2-(2-Tetrahydro-pyranyloxy)äthoxymethyl/-$N^2$-azetylguanin | 135-136,5 | 0,66 | 51,1 | 6,0 | 19,7 |
| 9-/2-(2-Tetrahydro-pyranyloxy)äthoxy-methyl/guanin | 202-204 | 0,44 | 50,3 | 6,3 | 22,4 |

Fortsetzung der Tabelle 1

| Verbindung | Bruttoformel | Berechnet in % C | H | N |
|---|---|---|---|---|
| 1 | 7 | 8 | 9 | 10 |
| 9-/2-(2-Tetra-hydrofuryloxy) äthoxymethyl/-$N^2$-azetylguanin | $C_{14}H_{19}N_5O_5$ | 49,8 | 5,7 | 20,8 |
| 9-/2-(2-Tetrahydro-furyloxy)äthoxy-methyl/-guanin | $C_{12}H_{17}N_5O_4 \cdot H_2O$ | 46,0 | 6,1 | 22,4 |
| 9-/2-(2-Tetra-hydropyranyloxy) äthoxymethyl/-$N^2$-azetylguanin | $C_{15}H_{21}N_5O_5$ | 51,3 | 6,0 | 19,9 |
| 9-(2-(2-Tetrahyd-ropyranyloxy)äthoxymethyl/guanin | $C_{13}H_{19}N_5O_4$ | 50,5 | 6,2 | 22,6 |

Tabelle 2. Spektrumparameter von 9-substituierten Guaninen

| Verbindung | UV-Spektrum in 0,01 Mol $H_3BO_3$ $\lambda_{max}/\varepsilon 10^{-3}$ / nm | NMR-Spektrum in Dimethylsulfoxid $d_6$ /Tetramethylsilan als Standard / $\delta$ ppm |
|---|---|---|
| 1 | 2 | 3 |
| 9-/2-(2-Tetrahydrofuryloxy)äthoxymethyl/-$N^2$-azetylguanin | 260/15,2/ 274/II,I/ | I2,0I, II,72 /NH/; 8,10/$H_8$/; 5,46/$NCH_2$/; 5,02/CH/; 3,60, I,76 /$CH_2$/; 2,I8 /$CH_3$/. |
| 9-/2-(2-Tetrahydrofuryloxy)äthoxymethyl/guanin | 253/I0,4/ 270/7,5/ | 10,59/NH/; 7,80/$H_8$/; 6,48 /$NH_2$/; 5,33/$NCH_2$/ 5,03/CH/; 3,63, I,76 /$CH_2$/. |
| 9-/2-(2-Tetrahydropyranyloxy)äthoxymethyl/-$N^2$-azetylguanin | 260/I4,9/, 276/I0,7/ | II,99, II,74/NH/; 8,I0/$H_8$/; 5,47 /$NCH_2$/; 4,49/CH/; 3,6I, I,46/$CH_2$/; 2,I8/$CH_3$/ |
| 9-/2-(2-Tetrahydropyranyloxy)äthoxymethyl/guanin | 253/I0,5/, 270/7,4/ | I0,57/NH/; 7,79/$H_8$/; 6,47/$NH_2$/; 5,34/$NCH_2$/; 5,II/CH/; 3,62, I,47 /$CH_2$/. |

## Die beste Ausführungsform der Erfindung

Es wurden Untersuchungen der antiviralen Aktivität der erfindungsgemäßen Verbindungen an Tieren im Vergleich zu Zovirax (Azyklovir) durchgeführt. Die Prüfungen erfolgten am Modell der experimentellen herpetischen Enzephalitis bei nicht reinrassigen weißen Mäusen. 8 bis 10 g schwere Mäuse wurden mit dem Herpes simplex-Virus des ersten Typs infiziert. Die Mäuse wurden auf zweierlei Art infiziert und zwar intraperitoneal in einer Dosis, die $LD_{50}$ hundertfach übersteigt, und intrazerebral in einer Dosis von 10 bis 30 $LD_{50}$. Die Substanzen wurden als wäßrige Lösungen oder Suspensionen mit Tween 80 intravenös und peroral verabreicht. Man benutzte folgende Methodik: Die erste Injektion erfolgte 3 Stunden nach der Infektion durch Virus und dann vom 2. bis den 7. Tag, bei intravenöser Verabreichung wurden die Verbindungen einmal täglich eingeführt, und bei peroraler Applikation wurde die Tagesdosis halbiert und zweimal täglich injiziert. Die Tagesdosen lagen zwischen 0,01 und 200 mg/kg. Zur peroralen Verabreichung löste man die Einheitsdosis in 0,1 ml destilliertem Wasser und zur intravenösen Verabreichung in 0,2 ml demselben auf.

Den Mäusen aus der Kontrollgruppe wurde das entsprechende Volumen der isotonischen Kochsalzlösung appliziert.

Die Wirksamkeit der Verbindungen wurde nach der Herabsetzung der Letalität bei Tieren, denen die erfindungsgemäßen Verbindungen und Zovirax (Vergleichspräparat) appliziert wurden, im Vergleich mit der Kontrollgruppe bewertet. Die Versuchsergebnisse sind in Tabellen 3 und 4 dargestelslt.

Tabelle 3. Herabsetzung der Letalität von durch Herpes simplex-Virus des Typs I intraperitoneal infizierten Mäusen mit herpetischer Enzephalitis im Falle der Verabreichung von 9-/2-(2-Tetrahydrofuryloxy)äthoxymethyl/-$N^2$-azetylguanin und Zovirax.

| Dosis der Substanz in mg/kg | 9-/2-(2-Tetrahydrofuryloxy)äthoxymethyl/-$N^2$-azetylguanin Letalität in % ± m | Herabsetzung der Letalität gegenüber Kontrolle in % | P |
|---|---|---|---|
| 1 | 2 | 3 | 4 |
| | Perorale Verabreichung der Verbindung | | |
| 200 | 25,0 | 60,0 | |
| I00 | I8,7 | 66,3 | |
| I0 | I0,0 | 75,0 | < 0,0I |
| I | 20,0 | 65,0 | > 0,05 |
| 0,I | 30,0±I5,3 | 55,0 | |
| 0,0I | 60,0±I6,3 | 25,0 | |
| Kontrolle | 85,7±8,2 | | |
| | Intravenöse Injektion der Verbindung | | |
| I00 | II,I | 69,8 | |
| I0 | 5,5 | 75,4 | |
| I | I2,5 | 68,4 | < 0,00I |
| 0,I | 22,2±I0,I | 58,7 | > 0,05 |
| 0,0I | 60,0±I6,3 | 20,9 | |
| Kontrolle | 80,9±8,8 | | |

Fortsetzung der Tabelle 3

| Dosis der Substanz in mg/kg | Zovirax (Azyklovir) Letalität in % ± m | Herabsetzung der Letalität gegenüber Kontrolle in % | P |
|---|---|---|---|
| 1 | 5 | 6 | 7 |
| | Perorale Verabreichung der Verbindung | | |
| 200 | 43,7 | 47,9 | |
| I00 | 33,3 | 38,3 | |
| I0 | 28,9 | 62,7 | |
| I | 42,I | 49,5 | < 0,00I |
| 0,I | 57,8±8,II | 33,8 | > 0,05 |
| 0,0I | 83,3±II,2 | 8,3 | |
| Kontrolle | 9I,6±4,0 | | |
| | Intravenöse Injektion der Verbindung | | |
| I00 | - | - | |
| I0 | 25,0 | 65,9 | |
| I | 40,9 | 50,0 | |
| 0,I | 59,I±I0,7 | 3I,8 | < 0,05 |
| 0,0I | 72,7±9,7 | 18,2 | > 0,05 |
| Kontrolle | 90,9±6,3 | | |

Wie aus der Tabelle 3 zu ersehen ist, setzt 9-/2-(2-Tetrahydrofuryloxy)äthoxymethyl/-$N^2$-azetylguanin die Letalität bei Mäusen sowohl im Falle der peroralen Verabreichung als auch bei der intravenösen Injektion herab. In beiden Fällen ist die Wirksamkeit gleich. Dies ist vielleicht damit verbunden, daß die Verbindung in einer gleichen Tagesdosis peroral zweimal und intravnös einmal täglich appliziert wurde.

Bei der zweimaligen Applikation der Verbindung innerhalb von 24 Stunden wurde die Wirksamkeit der peroralen Therapie erhöht und dabei wurde sie der der intravenösen Therapie herangebracht. Wie die Tabelle zeigt, ist die Wirksamkeit der erfindungsgemäßen Verbindung (die maximale Herabsetzung der Letalität um 75%) höher als die von Zovirax (die Verminderung der Letalität um 65,9%). Im Laufe der ähnlichen Versuche betrug die Wirksamkeit von 9-/2-(2-Tetrahydrofuryloxy)äthoxymethyl/guanin etwa 63 %, die von 9-/2-(2-Tetrahydropyranyloxy)äthoxymethyl/guanin etwa 58% und von 9-/2-(2-Tetrahydropyranyloxy)äthoxymethyl/-$N^2$-azetylguanin etwa 15 %.

Die Wirksamkeit von 9-/2-(2-Tetrahydrofuryloxy) äthoxymethyl/-$N^2$-azetylguanin ist auch an Mäusen, infiziert durch Herpes simplex-Virus intrazerebral, d.h. durch unmittelbare Verarreichung von Virus ins Hirn, geprüft. Die Virusdosis war 10 bis 30mal so groß wie $LD_{50}$ (die Prüfergebnisse sind in der Tabelle 4 dargestellt). Die Verbindungen wurden peroral und intravenös nach der im vorangegangenen Versuch angegebenen Methodik appliziert.

Wie aus der Tabelle 4 ersichtlich ist, ist die erfindungsgemäße Verbindung in einem breiteren Dosisspielraum wirksam.

Bei der peroralen Verabreichung der erfindungsgemäßen Verbindung war die maximale Wirkung (die Herabsetzung der Letalität um 56,3% gegenüber Kontrolle) im Falle der Einführung der Tagesdosis von 100 bzw. 200 mg/kg zu vermerken. Zovirax, appliziert in einer Tagesdosis von 100 mg/kg. und 200 mg/kg, verminderte die Letalität um 33,3 bzw. nur um 26,2 %. Bei geringeren Dosen (von 0,1 bis 10 mg/kg) war die Wirksamkeit der beiden Verbindungen ähnlich.

Bei der intravenösen Injektion der erfindungsgemäßen Verbindung in einer Dosis von 10 mg/kg wurde

die Letalität um 55,8 % herabgesetzt, was der Wirkung von Zovirax, appliziert in der gleichen Dosis, ent - spricht. Bei Zovirax kann man jedoch seine Dosis nicht vergrößern, ohne daß der pH-Wert der Lösung verändert wird, weil die Löslichkeit von Zovirax beschränkt ist. Falls 9-/2-(2-Tetrahydrofuryloxy)äthoxymethyl/-$N^2$-azetylguanin zur Verwendung kommt, kann seine Dosis auf 100 mg/kg vermehren. Die Letalität wird dadurch um 66,5% herabgesetzt. Die Breite der pharmakologischen Wirkung bei der erfindungsgemäßen Verbundung ist um das 10fache höher als beim Zovirax.

Im Laufe von in vitro-Versuchen unter Verwendung der einschichtigen Kultur der primären Fibroblasten von durch Herpes simplex-Virus des Typs I infizierten Hühnerembryos wurde die Anzahl von Platten gezählt. Die Senkung des Virustiters um über 1,5 lgBFU /ml im Falle der Verwenung von 9-/2-(2-Tetrahydrofuryloxy)äthoxymethyl/-$N^2$-azetylguanin, 9-/2-(2-Tetrahydrofuryloxy)-äthoxymethyl/-guanin und 9-/2-(2-Tetrahydropyranyloxy) äthoxymethyl/guanin fand man bei einer Konzentration von 100 µg/ml, während dies im Falle der Applikation von 9-/2-(2-Tetrahydropyranyloxy)äthoxymethyl/-$N^2$-azetylguanin bei einer Konzentration von 400 µg/ml statthat. Diese Konzentrationen liegen bedeutend höher als die minimale wirksame Konzentration von Zovirax. Da in vivo-Versuche eine hohe antivirale Aktivität der erfindungsgemäßen Verbindungen zeigen, kann man annehmen, daß sie im Organismus aktiviert werden, d.h. sie wirken als Transportformen, indem sie im Organismus in die aktive Verbindung umgesetzt werden. Damit is wahrscheinlich eine geringere als bei Zovirax toxische Gesamtwirkung der erfindungsgemäßen Verbindungen auf den Organismus von Tieren zu erklären.

So zum Beispiel beträgt $LD_{50}$ für Mäuse bei ein - maliger intraperitonealer Injektion von 9-/2-(2-

Tabelle 4. Herabsetzung der Letalität von durch Herpes simplex-Virus intrazerebral infizierten Mäusen im Falle der Verabreichung von 9-/2-(2-Tetrahydrofuryloxy)äthoxymethyl/-$N^2$-azelyguanin und Zovirax

| Dosis der Substanz in mg/kg | 9-/2-(2-Tetrahydrofuryloxy) äthoxymethyl/-$N^2$-azetylguanin Letalität in % ± m m | Herabsetzung der Letalität gegenüber Kontrolle in % | P |
|---|---|---|---|
| 1 | 2 | 3 | 4 |
| | Perorale Verabreichung der Verbindungen | | |
| 200 | 25,0 | 56,3 | |
| I00 | 25,0 | 56,3 | <0,05 |
| IO | 37,5 | 43,8 | >0,05 |
| I,0 | 50,0 | 31,3 | |
| 0,I | 50,0±I8,9 | 31,3 | |
| 0,0I | 75,0±I6,4 | 6,3 | |
| Kontrolle | 81,3±IO,I | | |
| | Intravenöse Injektion der Verbindungn | | |
| I00 | I4,3 | 66,5 | |
| IO | 25,0 | 55,8 | |
| I,0 | 37,5 | 43,5 | <0,02 |
| 0,I | 41,6±14,99 | 39,2 | > 0,05 |
| 0,0I | 91,4±8,3 | 0,0 | |
| Kontrolle | 90,8±7,9 | | |

Fortsetzung der Tabelle 4

| Dosis der Substanz in mg/kg | Zovirax (Azyklovir) Letalität in % ± m | Herabsetzung der Letalität gegenüber Kontrolle in % | P |
|---|---|---|---|
| 1 | 5 | 6 | 7 |
| | Perorale Verabreichung der Verbindungen | | |
| 200 | 57,I | 26,2 | |
| IOO | 50,0 | 33,3 | <0,03 |
| IO | 28,5 | 54,8 | >0,05 |
| I,0 | 50,0 | 33,3 | |
| 0,I | 55,0±II,4 | 28,3 | |
| 0,0I | 70,0±IO,5 | 13,3 | |
| Kontrolle | 83,3±6,3 | | |
| | Intravenöse Injektion der Verbindungen | | |
| IOO | - | | |
| IO | 33,3 | 55,2 | <0,05 |
| I,0 | 43,7 | 44,8 | >0,05 |
| 0,I | 64,3±I3,3 | 24,2 | |
| 0,0I | 78,5±II,4 | IO,0 | |
| Kontrolle | 88,5±6,4 | | |

-Tetrahydrofuryloxy)äthoxymethyl/-$N^2$-azetylguanin 3300 mg/kg, bei 9-/2-(2-Tetrahydropyranyloxy)äthoxymethyl/guanin übersteigt $LD_{50}$ 2000 mg/kg und ist 1800 mg/kg bei Zovirax gleich.

Die erfindungsgemäßen Verbindungen weisen eine bessere gegenüber Zovirax Wasserlöslichkeit auf. Besonders gut wasserlöslich ist 9-/2-(2-Tetrahydrofuryloxy)äthoxymethyl/-$N^2$-azetylguanin, seine Löslichkeit beträgt 120 mg/ml und übersteigt die Löslichkeit von Zovirax (2 mg/ml) auf das 60fache.

Die Löslichkeit von 9-/2-(2-Tetrahydropyranyloxy)äthoxymethyl/-$N^2$-azetylguanin liegt ebenfalls bei ca. 100 mg/ml. Die Löslichkeit von 9-/2-(2-Tetrahydrofuryloxy)-äthoxymethyl/-guanin und 9-/2-(2-Tetrahydropyranyloxy)-äthoxymethyl/guanin beträgt 2,5 bis 3 mg/ml. Die erfindungsgemäßen Verbindungen sind außerdem besser als Zovirax in schwach polaren organischen Lösungsmitteln löslich. So beträgt die Löslichkeit von 9-/2-(2-Tetrahydrofuryloxy)äthoxymethyl/-$N^2$-azetylguanin in Chloroform und die von Zovirax 10 mg/ml bzw. unter 1 mg/ml. Daraux kann man voraussetzen, daß 9-/2-(2-Tetrahydrofuryloxy)-äthoxymethyl/-$N^2$-azetylguanin in lipophile Substanzen des Organismus, darunter in Ganglien von sensiblen Nerven, wo die Ansammlung von Virusteilchen in der Latenzzeit der Krankheit erfolgt, tiefer eindringen kann.

Die erfindungsgemäßen Verbindungen sind in neutralen wäßrigen Lösungen (pH-Wert von etwa 7,4) beständig, werden jedoch in einem sauren Medium bei einem pH-Wert von weniger als hydrolysiert.

Die gewonnenen Ergebnisse zeigen die Vorteile der erfindungsgemäßen Verbindungen gegenüber Zovirax (Azyklovir):

a) eine bedeutend höhere (60fache) Wasserlöslichkeit, welche die Lösungen von 9-/2-(2-Tetrahydrofuryloxy)-

äthoxymethyl/-$N^2$-azetylguanin für intravenöse und intraperitoneale Injektionen zu verwenden ermöglicht;

b) eine geringere Nephrotoxizität und als Folge daraus eine Möglichkeit, in den Organismus höhere (10fache) Dosen einzuführen, was eine größere Herabsetzung der Letalität, bedingt durch herpetische Enzephalitis von Mäusen (um 66,5 % bei intrazerebraler Einführung von Virus und intravenöser Injektion der Verbindung und um 55 % bei Azyklovir) ergibt.

c) eine vollständigere Löslichkeit in schwach polaren organischen Lösungsmitteln (Azyklovir ist darin unlöslich), was das Eindringen in Ganglien sensibler Nerven ermöglicht, wo die Ansammlung von Virusteilchen in der Latenzzeit der Krankheit erfolgt.

Die erfindungsgemäßen Verbindungen sind wie folgt hergestellt.

In einen mit Rürwerk versehenen Kolben bringt man 9-(2-Azetoxyäthoxymethyl)-$N^2$-azetylguanin ein und setzt das Wasser zu. Die gewonnene Suspension rührt man um und kühlt auf eine zwischen 0 und minus 5°C liegende Temperatur ab, gibt dann die abgekühlte 2 n-NaOH-Lösung zu. Der Kolbeninhalt wird bei der gleichen Temperatur noch 1 Stunde lang umgerührt und mit Essigsäure bis zum Erzielen eines pH-Wertes von 6 bis 7 neutralisiert. Im Ergebnis wird eine Lösung gewonnen, die innerhalb von 24 Stunden bei einer zwischen 0 und minus 5°C liegenden Temperatur gehalten wird. Der ausgefallene Niederschlag von 9-(2-Hydroxyäthoxymethyl)-$N^2$-azetylguanin wird abfiltriert, gewaschen und getrocknet.

Der gewonnene 9-(2-Hydroxyäthoxymethyl)-$N^2$-azetylguanin wird bei einer Temperatur von 50 bis 60°C in Dimethylformamid aufgelöst. Die Lösung wird auf eine zwischen 0 und minus 5° liegende Temperatur abgekühlt und mit Chlorwasserstoff bei dieser Temperatur gesättigt. Hiernach setzt man unter Umrühren 2,3--Dihydrofuran oder 2,3-Dihydropyran zu. Das Reaktionsgut wird innerhalb von 3 Stunden bei einer zwischen 0 und minus 5°C liegenden Temperatur verwischt, wonach es mit Triäthylamin neutralisiert wird. Der ausgefallene Niederschlag von Triäthylaminhydrochlorid wird abfiltriert. Das Filtrat dampft man im Vakuum ein und den Rücksband kristallisiert aus Äthylazetat und dann aus Äthanol um. Man erhält 9-/2-(2-Tetrahydrofuryloxy)äthoxymethyl/-$N^2$-azetylguanin oder, falls man 2,3-Dihydropyran statt 2,3-Dihydrofuran verwendet, 9-/2-(2-Tetrahydropyranyloxy)äthoxymethyl/-$N^2$-azetylguanin.

Man gibt dem hergestellten 9-/2-(2-Tetrahydrofuryloxy)-äthoxymethyl/-$N^2$-azetylguanin eine wäßrige Lösung von Methylamin zu, vermischt innerhalb von 2 Stunden bei Raumtemperatur und dampft dann im Vakuum ein. Der Rückstand wird aus wäßririgem Äthanol umkristallisiert.

Man erhält 9-/2-(2-Tetrahydrofuryloxy)äthoxymethyl/-guanin.

Behandelt man 9-/2-(2-Tetrahydropyranyloxy)äthoxymethyl/-$N^2$-azetylguanin mit wäßriger Methylaminlösung, so erhält man auf ähnlicher Weise 9-/2-(2-Tetrahydropyranyloxy)äthoxymethyl/guanin.

Zum besseren Verstehen der vorliegenden Erfindung werden folgende konkrete Beispiele angeführt.

Beispiel 1

In einen mit Rührwerk versehenen Kolben bringt man 3,0 g (96 mMol) 9-(2-Azetoxymethyl)-$N^2$-azetylguanin und 60 ml Wasser ein. Die entstandene Suspension kühlt man auf 0°C ab und setzt unter Umrühren 60ml 2 n-NaOH-Lösung zu, die im voraus auf 0°C abgekühlt worden ist. Das Reaktionsgut wird bei einer zwischen 0 und minus 5°C liegenden Temperatur innerhalb von 1 Stunde umgerührt und dann mit der Essigsäure bis zum Erzielen eines pH-Wertes von 6 bis 7 neutralisiert. Nach der Abkühlung innerhalb von 24 Stunden wird der ausgefallene Niederschlag

abfiltriert, mit einer geringen Kaltwassermenge gewaschen und getrocknet. Man erhält 2,18 g (84%) 9-(2-Hydroxyäthoxymethyl)-$N^2$-azetylguanin mit Schmelzpunkt von 212 bis 214°C, $R_f$ = 0,21 (im Chloroform-Methanol-System beim Verhältnis von 10:2).

1,20 g (4,5 mMol) 9-(2-Hydroxyäthoxymethyl)-$N^2$-azetylguanin, hergestellt wie oben beschrieben, wird in einen mit Rührwerk versehenen Kolben eingebracht und bei einer zwischen 50 und 60°C liegenden Temperatur in 40 ml Dimethylformamid aufgelöst. Die Lösung wird auf eine zwischen 0 und 5°C liegende Temperatur abgekühlt und bei dieser Temperatur mit trockenem Chlorwasserstoff gesättigt. Dann werden dem Reaktionsgut 4 ml 2,3-Dihydrofuran unter Umrühren und Abkühlung auf 0°C allmählich hinzugefügt. Man vermischt die Lösung innerhalb von 3 Stunden bei einer zwischen 0 und minus 5°C liegenden Temperatur und neutralisiert danach den überschüssigen Chlorwasserstoff mit Triäthylamin.

Der ausgefallene Niederschlag von Triäthylaminhydrochlorid wird abfiltriert und mit einer geringen Menge von Dimethylformamid gewaschen. Das Filtrat dampft man im Vakuum ein. Der Rückstand wird aus trockenem Äthylazetat und dann aus Äthanol umkristallisiert. Man stellt 1,26 g (82,9%) 9-/2-(2-Tetrahydrofuryloxy)äthoxymethyl/-$N^2$-azetylguanin her.

Die physikalisch-chemischen Kenndaten, welche die Struktur der Verbindung nachweisen, sind in den oben angegebenen Tabellen 1 und 2 zusammengestellt.

Beispiel 2

In einen mit Rührwerk versehenen Kolben bringt man 1,18 g (3,5 mMol) 9-/2-(2-Tetrahydrofuryloxy)äthoxymethyl/-$N^2$-azetylguanin, hergestelslt wie in Beispiel 1 beschrieben, ein. Man gibt 20 ml 25%ige wäßrige Lösung von Methylamin zu. Das Reaktionsgut wird bei Raumtemperatur innerhalb von 2 Stunden umgerührt und

danach im Vakuum bei einer 30°C nicht übersteigenden Temperatur eingedampft. Der trockene Rückstand wird zweimal aus wäßrigem Äthanol umkristallisiert. Man erhält 0,87 g (84 % ) 9-/2-(2-Tetrahydrofuryloxy)äthoxymethyl/-guanin.

Beispiel 3

In einen mit Rührwerk versehenen Kolben bringt man 1,20 g (4,5 mMol) 9-(2-Hydroxyäthoxymethyl)-$N^2$-azetylguanin, hergestellt wie in Beispiel 1 beschrieben, ein. Man setzt 40 ml Dimethylformamid zu und erwärmt bei einer Temperatur von 50 bis 60°C, bis der Niederschlag vollständig aufgelöst wird. Die gewonnene Lösung wird auf eine zwischen 0 und minus 5°C liegende Temperatur abgekühlt und bei dieser Temperatur mit trockenem Chlorwasserstoff, gesättigt. Hiernach werden dem Reaktionsgut 4 ml 2,3-Dihydropyran unter Ümrühren und Abkühlung auf 0°C allmählich hinzugefügt, wonach man den überschüssigen Chlorwasserstoff durch Zugabe von Triäthylamin neutralisiert. Man filtriert den ausgefallenen Niederschlag von Triäthylaminhydrochlorid ab und dampft das Filtrat im Vakuum ein. Der Rückstand wird aus trockenem Äthylazetat und dann aus Äthanol umkristallisiert. Man erhält 0,89 g (56,5 %) 9-/2-(2-Tetrahydropyranyloxy)äthoxymethyl/-$N^2$-azetylguanin.

Beispiel 4

In einen mit Rührwerk versehenen Kolben bringt man 1,23 g (3,5 mMol) 9-/2-(2-Tetrahydropyranyloxy)äthoxymethyl/-$N^2$-azetylguanin, hergestellt wie in Beispiel 3 beschrieben, ein. Man setzt 20 ml 25%ige wäßrige Lösung von Methylamin zu. Das Reaktionsgut wird bei Raumtemperatur innerhalb von 2 Stunden umgerührt und dann bei einer 30°C nicht übersteigenden Temperatur im Vakuum eingedampft. Der trockene Rückstand wird zweimal aus wäßrigem Äthanol umkristallisiert. Man erhält 0,86 g (80 %) 9-/2-(2-Tetra-

hydropyranyloxy)äthoxymethyl/-guanin.

Die physikalisch-chemischen Eigenschaften der in Beispielen 1 bis 4 hergestellten Verbindungen, welche ihre Struktur nachweisen, sind in den Tabellen 1 und 2 zusammengestellt.

Industrielle Anwendbarkeit

Die erfindungsgemäßen Verbindungen können in der Medizin Verwendung finden.

PATENTANSPRUCH

9-substituierte Guanine der allgemeinen Formel

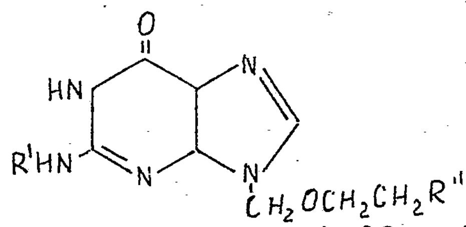

worin R' für Azetyl, Wasserstoff und R'' für 2-Tetrahydrofuryl, 2-Tetrahydropyranyl stehen.

# INTERNATIONAL SEARCH REPORT

International Application No PCT/SU 87/00195

**I. CLASSIFICATION OF SUBJECT MATTER** (if several classification symbols apply, indicate all) [6]

According to International Patent Classification (IPC) or to both National Classification and IPC

$IPC^4$ C 07 D 473/18, A 61 K 31/52

**II. FIELDS SEARCHED**

Minimum Documentation Searched [7]

| Classification System | Classification Symbols |
|---|---|
| $IPC^4$ | C 07 D 473/18, A 61 K 31/52 |

Documentation Searched other than Minimum Documentation to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| A | GB, A, 2122197, (Astra Lakemedel Aktiebolag), 11 January 1984 (11.01.84), see the abstract | 1 |
| A | GB, A, 2122198, (Astra Lakemedel Aktiebolag), 11 January 1984 (11.01.84), see the abstract | 1 |
| A | US, A, 4612314, (Syntex (U.S.A.)Inc.), 16 September 1986 (16.09.86), see the abstract | 1 |
| A | WO, A1, 84/00167, (ASTRA LÄKEMEDEL AKTIEBOLAG), 19 January 1984 (19.01.84), see the abstract | 1 |
| A | US, A, 4507305, (Syntex (U.S.A.)Inc.), 26 March 1985 (26.03.85), see the abstract | 1 |
| A | EP, B1, 0103551, (Astra Läkemedel Aktiebolag), 26 February 1986 (26.02.86), see the claims | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 8 February 1988 (08.02.88) | 10 March 1988 (10.03.88) |
| **International Searching Authority** | **Signature of Authorized Officer** |
| ISA/SU | |